# EUROPEAN PATENT SPECIFICATION

(11) **EP 0 462 549 B1**
(45) Date of publication and mention of the grant of the patent: **28.08.1996**
(21) Application number: 91109923.2
(22) Date of filing: 18.06.1991
(51) Int. Cl.: C07K 2/00, A61K 38/00

(54) **Process of preparing a therapeutic agent for renal diseases**
Verfahren zur Herstellung eines therapeutischen Mittels für Nierenkrankheiten
Procédé pour la préparation d'un agent thérapeutique pour les maladies rénales

(30) Priority: 19.06.1990 JP 158841/90
(43) Date of publication of application: 27.12.1991
(73) Proprietor: NAKAMURA, Toshikazu, Takatsuki-shi, Osaka 569 (JP)
(72) Inventor: Nakamuro, Toshikazu, Fukuoka-shi, Fukuoka 812 (JP)
(74) Representative: von Kreisler, Alek, Dipl.-Chem.

(56) References cited:
- EP-A- 0 412 557
- BIOCHEMICAL & BIOPHYSICAL RESEARCH COMMUNICATIONS, vol. 174, no. 1, 15 January 1991, US; M. KAN et al., pp. 331-337
- BIOCHEMICAL & BIOPHYSICAL RESEARCH COMMUNICATIONS, vol. 174, no. 2, 31 January 1991, US; T. IGAWA et al., pp. 831-838
- NATURE, vol. 342, no. 6248, 23 November 1989, London (GB); T. NAKAMURA et al., pp. 440-443
- PATENT ABSTRACTS OF JAPAN, vol. 12, no. 230 (C-508)(3077), 29 June 1988
- PATENT ABSTRACTS OF JAPAN, vol. 11, no. 232 (C-437)(2679), 29 July 1987
- PATENT ABSTRACTS OF JAPAN, vol. 15, no. 60 (C-805)(4588), 13 February 1991
- PATENT ABSTRACTS OF JAPAN, vol. 9, no. 175 (C-292)(1898), 19 July 1985
- PATENT ABSTRACTS OF JAPAN, vol. 13, no. 174 (C-589)(3522), 25 April 1989
- PATENT ABSTRACTS OF JAPAN, vol. 13, no. 208 (C-596)(3556), 16 May 1989

## Description

### BACKGROUND OF THE INVENTION

The present invention relates to the use of a hepatocyte growth factor (HGF) for the production of a therapeutic and a preventive agent for renal diseases of human or mammalian animals.

Kidney is an aggregate of about one million functional units called nephron and is an organ in control of excretory function by urine production. Also, the kidney is an organ having a wide range of physiological functions such as regulation of electrolytes (e.g. sodium ion and calcium ion) in body fluid (e.g. blood), osmotic pressure and body fluid, and as an endocrine organ, control over differentiation and maturation of erythroid cells by producing erythropoietin which is a kind of hormone and activation of 25-hydroxy vitamin D which is a precursor of vitamin D.

Nephron constituting the kidney comprises glomerulus and renal tubules wherein glomerulus filters blood, and renal tubules reabsorb necessary substance from the glomerulus filtrate and discharge unnecessary substance. The renal tubules consist of proximal tubule, Henle's tubule, distal tubule and collecting tubulus. Among those, proximal tubule produces erythropoietin and activates vitamin D (Blood, *68* (Suppl.), 170a, 1986; Proc. Natl. Acad. Sci. USA, *28*, 1199, 1981) and regeneration of the kidney is known to be controlled by proximal tubular cells.

Renal diseases are largely classified into glomerulopathy in which lesion is observed in glomerulus, and tubulopathy in which lesion is observed in renal tubule(s). The causal disorder of chronic renal failure is said to be mostly the former and glomerulopathy is further classified into lupus nephritis (SLE), chronic pyelitis, IgA nephropathy, and so on. Diseases in which proteurea is a common symptom are also considered to be caused by disorder in glomerulus. Conventionally, steroid agents have been used for the treatment of nephritis. If chronic nephritis deteriorates into chronic renal failure for which dialysis is necessary, the function of the deteriorated kidney cannot be restored forever and continuous dialysis becomes indispensable through lifetime. At present, there are 300,000 patients with renal diseases in Japan, among which crucial patients undergoing dialysis treatment number in 90,000 with annual increase of about 8,000 patients. Although the single radical treatment of chronic renal failure is kidney transplantation, there are many problems in terms of donors, rejection reaction, and so on, and development of radical treatment methods or curative agents has been a strong demand.

Renal growth factor (RGF) which has been attracting great attention as a remarkable therapeutic agent for renal diseases was isolated from sheep in 1988 and its effect of growing proximal tubular cells of the kidney has been confirmed. In addition, epithelial cell growth factor (EGF) has been reported to show compensatory hypertrophy after nephrectomy of one member of the pair. Nevertheless, neither of them has been put to practical use.

In the meantime, hepatocyte growth factor which is an active ingredient of the present invention (hereinafter refferred to as HGF) is a protein discovered by the present inventors from serum of rats with regenerated liver as a factor which allows mature hepatocytes to grow *in vitro* (Biochem. Biophys. Res. Commun. *122*, 1450, 1984). The present inventors further succeeded in isolating HGF from rat platelet (Proc. Natl. Acad. Sci., *83*, 6489, 1986; FFBS Letter, *22*, 311, 1987) and determined its amino acid sequence partially. The present inventors conducted cloning of human- and rat-derived HGF cDNAs on the basis of the obtained HGF amino acid sequence, and succeeded in obtaining the hepatocyte growth factor as a protein by recombination of this cDNA using animal tissues (human HGF : Nature, *342*, 440, 1989; rat HGF : Proc. Natl. Acad. Sci, *87*, 3200, 1990).

Chronic nephritis comprises glomerulus nephritis beginning in disorder of glomerulus and tubular nephritis beginning in renal tubules, wherein the former involves damage of glomerulus and subsequent deterioration of renal tubules, and the latter involves only deterioration of renal tubules. Clinically important lupus nephritis (SLE) and IgA nephropathy are included in glomerulus nephritis, with their onset mechanism being immunological or non-immunological. As an example of the former mechanism, there has been known deposition of immuno complex (IC) in glomerulus, which comprises antigen derived from bacteria such as *Staphylococcus* and *Streptococcus haemolyticus* or virus of hepatitis B, measles, etc. and antibody against these. In addition thereto, other various causes are considered to be present, leaving much to be clarified. Steroid agents are used for acute nephritis and chronic nephritis in earlier stages. However, if chronic nephritis proceeds into renal failure, there is no radical treatment to be offered but dialysis. Nevertheless, secretion of hormones, etc. from the kidney is very important in functional terms, and specifically, decrease of erythropoietin due to deterioration of proximal tubule which produces it causes critical anemia. Although it has been confirmed that administration of EGF solely or in combination with insulin promotes proliferation of proximal tubular cells and compensatory hypertrophy of the kidney, EGF potently promotes proliferation of carcinoma cells, too, which encounters many problems in practical use. Thus, a therapeutic agent which promotes proliferation of nephrocytes along with insulin but causes no such side effects has been desired.

Persistent chronic nephritis stagnates discharge of waste product in body fluid, increasing load on the kidney, and encourages progress of nephritis and deterioration. While the kidney with renal failure where glomerulus and renal tubules are completely deteriorated never regenerate by itself, there is no treatment methods nor drugs capable of regenerating the kidney available at present. Kidney transplantation is conducted as a means of remedy for patients who must rely on dialysis through their life, there are not many actual demonstrations due to shortage of donors in absolute number and difficulty in histocompatibility. Nephrectomy or transplantation of the kidney is also adopted as a radical treatment method for carcinoma, etc. In this case, compensatory hypertrophy of the transplanted kidney or the residual kidney after nephrectomy of one member of the pair is essential for patients, but an agent promoting such activities has not been developed yet.

Administration of drugs such as an antibiotic increases load on the kidney in normal humans as well as in patients with renal diseases and sometimes induces renal hypofunction. For this reason, it is important to prevent induction of renal disorders caused by drugs.

Kidney is an organ which produces various hormones such as erythropoietin and enzymes. Although artificial cultivation of nephrocytes *in vitro* is significant for production of biological factors as pharmaceuticals, a pharmaceutical possessing nephrocyte growing activity is not available at present.

As a means of renal function diagnosis, there have been employed measurement of protein and/or sugar in urea. Unlike such means, the present invention aims at diagnosis of the phase of inflammation and function recovery of the kidney by assaying HGF, which is clinically very useful.

### SUMMARY OF THE INVENTION

The object of the present invention is to provide a therapeutic agent capable of promoting nephrocyte growth, kidney regeneration in chronic nephritis and compensatory hypertrophy of the kidney; an agent capable of ameliorating renal failure; and a preventive for renal disorders caused by drugs.

The present invention provides the use of a hepatocyte growth factor (HGF) for the production of a therapeutic agent for renal diseases of human or mammalian animals and further provides the use of a hepatocyte growth factor (HGF) for the production of a preventive agent for renal diseases of human or mammalian animals.

That is, the present inventors have conducted intensive studies for the purpose of solving the aforementioned problems and newly found that HGF was produced in the kidney when the residual kidney of a rat from which the other kidney had been removed showed compensatory hypertrophy. Taking note of such finding, the present inventors have found that HGF has promoting activities on growth of nephrocytes such as proximal tubular cells of the kidney and mesangial cells, and regeneration of the kidney, which resulted in completion of the invention. Particularly, proximal tubular cells initiate regeneration of the kidney and secrete important hormones such as erythropoietin, and thus, the growth promotion of proximal tubular cells by HGF is critical to the amelioration of renal diseases. In addition, HGF exhibits growth activity on hepatocytes in the body with hepatic diseases, while it does not induce side effects such as hypertrophy or hypofunction of the liver when administered to bodies with normal liver, which confirms excellent properties as a therapeutic agent for renal diseases. HGF of the present invention promotes compensatory hypertrophy of the kidney, and can be advantageously used as an agent for improving renal function after nephrectomy or kidney transplantation. Also, co-administration of HGF with a medicament such as an antibiotic for treatment purposes is confirmed to have preventive effect on renal disorders.

### BRIEF EXPLANATION OF THE DRAWINGS

Fig. 1 shows the amino acid sequence of human HGF and DNA sequence coding for the same. The base sequence is given in the upper space and the amino acid sequence is given in the lower space of a line.

Fig. 2 is a graph showing the growth-promoting activity of rat HGF on rat proximal tubular cells. 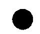 means that an HGF is added and ▲ means that EGF + insulin is added.

### DETAILED DESCRIPTION OF THE INVENTION

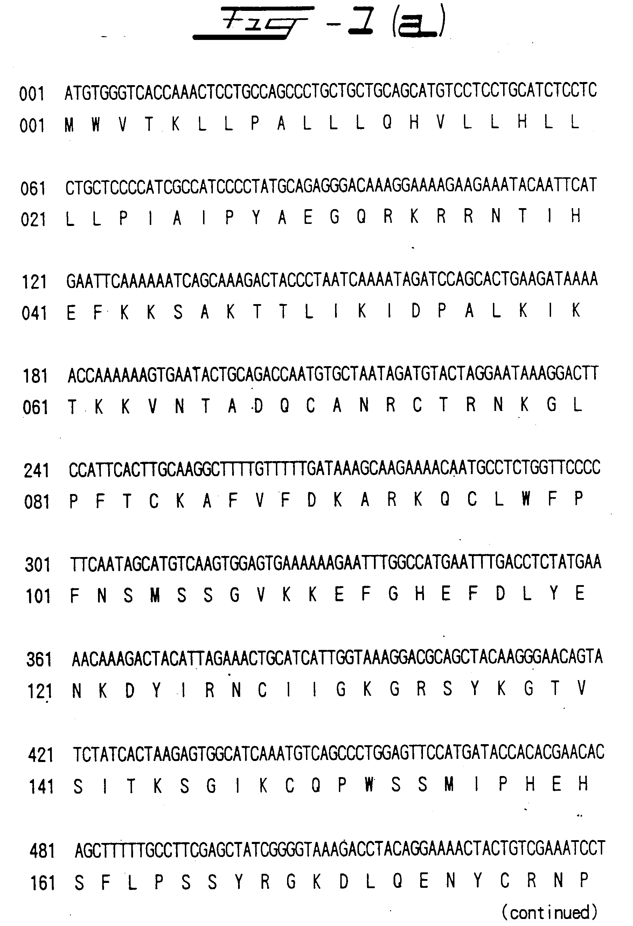

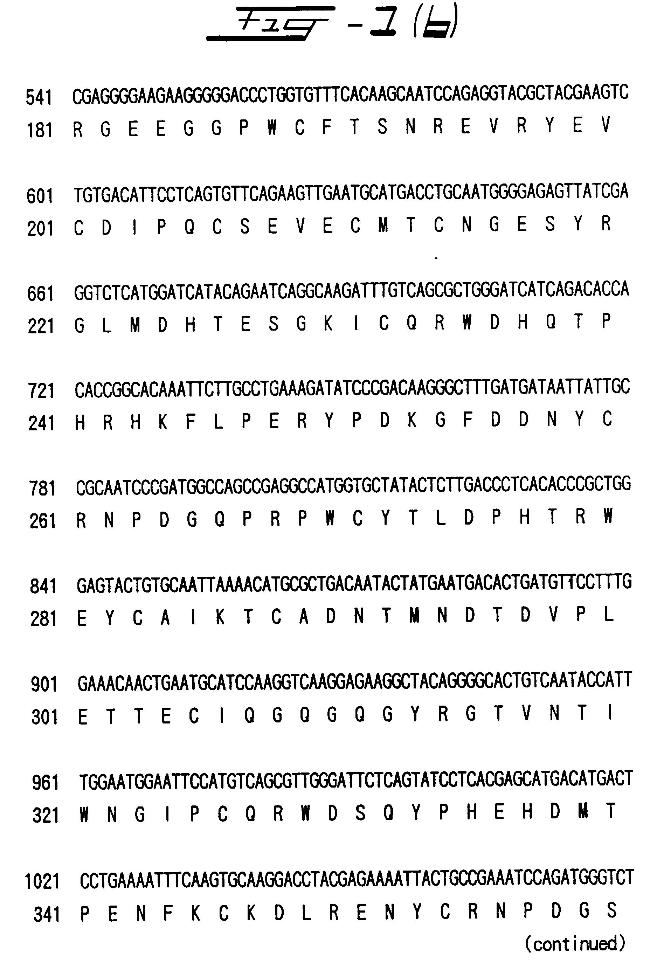

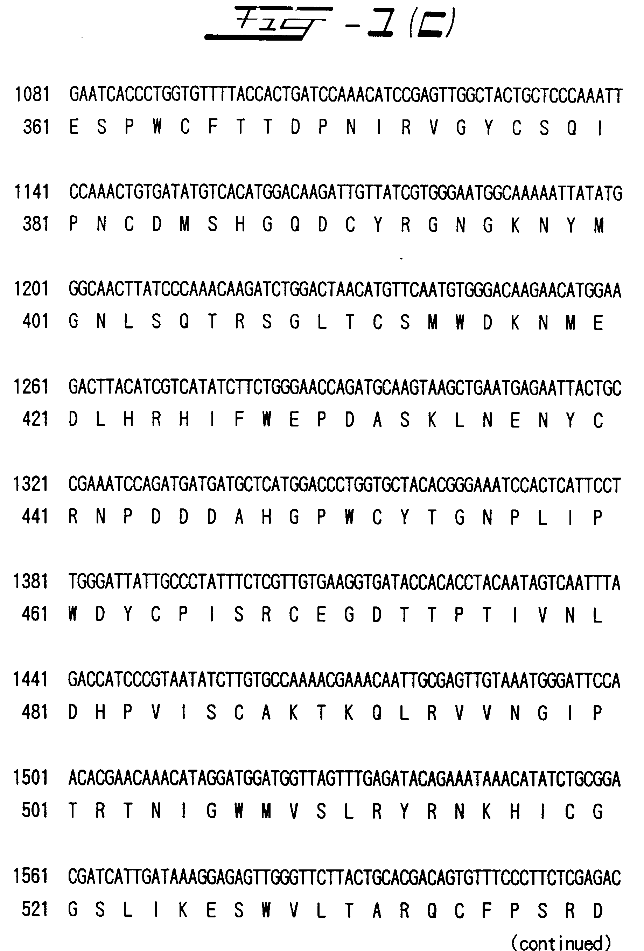

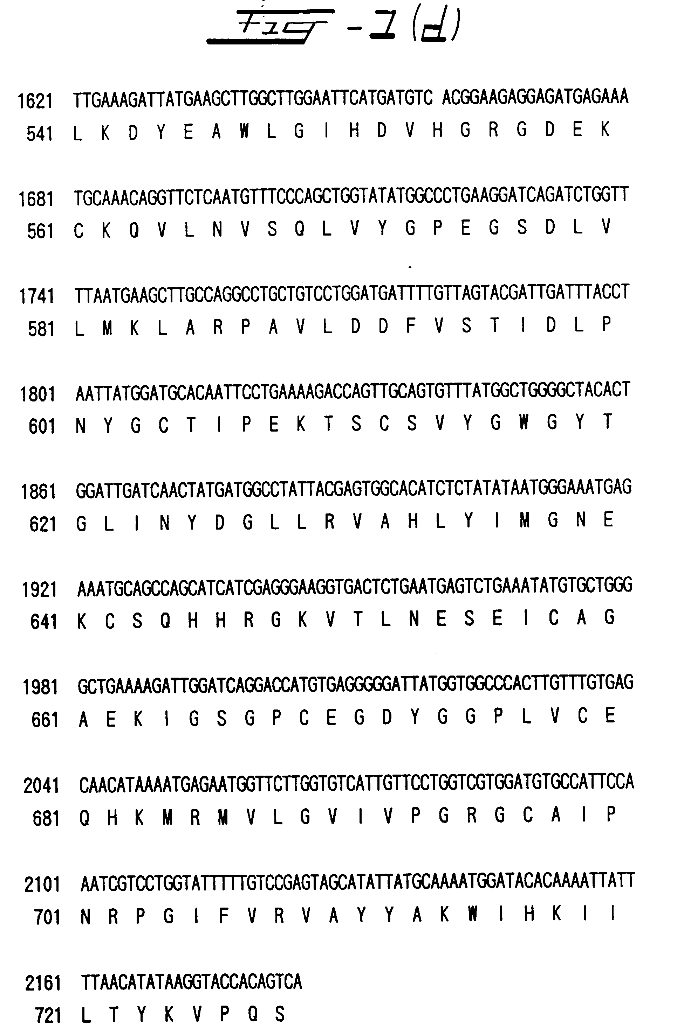

HGF used in the present invention is a physiologically active polypeptide discovered as a factor capable of growing mature rat hepatocytes *in vitro,* which has 82 to 85 kD molecular weight by SDS-polyacrylamide gel electrophoresis. Rat HGF has the heterodimer structure comprising α-chain of 440 amino acid residues and β-chain of 233 amino acid residues cross-linked by a disulfide bond, the both of which having 2 glucosamine sugar chain bonding sites. Human HGF also has almost the same physiological activity and comprises α-chain of 440 amino acid residues and β-chain of 234 amino acid residues. In the α-chain, there are 4 kringle structures similar to that of plasmin and the amino acid sequence of the β-chain has about 37% homology with β-chain of plasmin having serine protease activity. The amino acid sequence of the human HGF and the base sequence coding for it are shown in Fig. 1. Homology of the amino acid sequence of rat HGF and human HGF is 91.6% in the α-chain and 88.9% in the β-chain, and their activities have non-species specificity.

That a hepatocyte growth factor is useful as a therapeutic and preventive agent for renal diseases and an agent for nephrocyte growth, and can be used as a diagnostic for renal diseases was found for the first time by the present inventors.

HGF of the present invention can be produced by various methods. For example, it can be obtained from organs such as liver, spleen, lung, bone marrow, brain, kidney and placenta, blood cells such as platelet and leukocyte, plasma and serum of mammals such as rat and cow by extraction and purification. It is also possible to obtain HGF by cultivation of primary culture cells and strain cells capable of HGF production, followed by separation and purification from the culture. Or HGF can be obtained by genetic engineering comprising isolation of the HGF gene and transformation of suitable host cells such as *Escherichia coli, Bacillus subtilis,* yeasts, filamentous fungi, plant or animal cells to give the objective recombinant hepatocyte growth factor from the culture of the transformants thereof (Nature, *342*, 440, 1989).

The thus-obtained HGF can be used in the present invention as long as it has growth activity for nephrocytes such as proximal tubular cells even if the amino acid sequence is partially deleted and/or substituted, or other amino acid sequence is partially inserted, or sugars are similarly deleted and/or substituted.

HGF which is the active ingredient of the agent of the present invention has excellent growth activity for nephrocytes irrespective of from which of the mammals such as human, cow, horse, rat, sheep it is derived, and shows effective nephrocyte growth activity to all mammals. That is, the therapeutic agent, and so on for renal diseases of the present invention can be used as a pharmaceutical for not only humans but also for animals.

The therapeutic and preventive agent of the present invention is generally injected with HGF alone as the active ingredient or in combination with other known carriers. For example, injections can be prepared by dissolving HGF in a suitable buffer, filtering through a filter and aseptically filling in a container. The therapeutic and preventive agent of the present invention may contain with HGF additives necessary for formulation, such as stabilizers, excipients, dissolution-promotors and antioxidants. The liquid preparations are preferably stored by cryopreservation or after removal of water content by lyophilization.

The therapeutic agent and the preventive of the present invention are administered via suitable administration route according to the form of the preparation composition. For example, they can be administered in the form of injections intravenously, intra-arterially, subcutaneously or intramuscularly. The dose amount ranges from 0.1 mg to 100 mg by the amount of HGF, which can be administered singly or in several times divided doses a day.

The therapeutic and preventive agent of the present invention contains HGF as an active ingredient and utilizes nephrocyte growth activity of HGF on mesangial cells such as proximal tubular cells, thus promoting regeneration of nephrocytes in chronic nephritis and preventing transition to renal failure, while promoting regeneration of the kidney with renal failure and recovering renal functions to a normal state. Thus, they are extremely useful as a therapeutic and preventive agent for renal diseases for which there have been no efficacious treatment methods.

The present invention is hereinbelow described in detail by illustrating working examples so as to clarify embodiment and effects of the invention, which are not to be construed as limitative.

### Example 1

The hepatocyte growth factor HGF of the invention was produced from a rat liver as in the following.

Carbon tetrachloride (0.2% body weight of rat) was intraperitoneally administered to Wister rat, and 30 hours later, the rat liver was removed. The liver was homogenized by a whirling blender, after which it was centrifuged at 10,000 rpm for 20 minutes with a Hitachi 20 PR-52 cooling centrifuge to give the supernatant. The supernatant was dialyzed against a mixed solution of 50 mM tris hydrochloric acid buffer (pH 8.5), 0.15 M NaCl, 10 mM HEPES, 2 mM CaCl₂ and 0.01% Tween 80 at 4°C for a whole day. The dialyzed solution was poured onto an S-Sepharose (FF, Pharmacia) column equilibrated with dialyzing fluid, and after washing, it was eluted with the gradient of NaCl. The hepatocyte growth factor was eluted at about 0.7 M NaCl concentration. This HGF was then purified by Blue Tris acryl M (IBF Corp. ) chromatography. Elution was conducted with the gradient of arginine, and the HGF was eluted at about 0.25 M arginine concentration. The obtained fraction was then purified by heparin-Sepharose (Pharmacia) chromatography. Elution was conducted with the gradient of NaCl, and the HGF was eluted at about 1 M NaCl concentration, which was then purified by phenyl 5PW (Toso Corp.) chromatography. Elution was conducted with the decrease gradient of NaCl and the increase gradient of ethylene glycol, and 10 µg of HGF was obtained from livers of 100 rats. Relative activity of the HGF was about 500,000 unit/mg. To the obtained HGF was added 0.25% BSA (bovine serum albumin) and the mixture was dialyzed with PBS (phosphate buffer saline).

### Example 2

Using genetic engineering, human cell-derived hepatocyte growth factor HGF was produced.

In accordance with the Wigler et al method (Cell, *11*, 223, 1977), mouse C127 cells transformed with a gene coding for the amino acid sequence of the human hepatocyte growth factor were cultured, and the human hepatocyte growth factor was obtained from the culture supernatant thereof. That is, a cDNA library prepared from mRNA of human liver was subjected to screening, by which clone HAC19 and clone HBC25 coding for the amino acid sequence of the human hepatocyte growth factor were obtained.

DNAs from the HAC19 and the HBC25 were digested with BamHI and ScaI, and ScaI and PstI, respectively. The thus-obtained two DNA fragments were ligated with Blue Script KSII at BamHI and PstI sites to obtain pBS[hHGFII] (FERM BP-2990 deposited at the Fermentation Research Institute, Agency of Industrial Science and Technology, Ministry of International Trade and Industry, Japan). The pBS[hHGFII] was digested with XbaI, SalI and NaeI, and given blunt ends by T4 DNA polymerase, after which an about 3 Kb DNA fragment coding for the human hepatocyte growth factor was inserted at EcoRV site of an expression vector pBPMT prepared with bovine papilloma virus DNA as a vector, to give pBPMT[hHGFII]. The thus-obtained hepatocyte growth factor expression vector pBPMT[hHGFII] was used to transform mouse C127 cells by the calcium phosphate method. Selection of the transformants was conducted by growth thereof in a medium containing G418. The cell line BPH89 showing high hepatocyte growth factor producing activity was selected from among the transformants obtained. After the BPH89 cells were grown in a medium supplemented with fetal calf serum, the medium was exchanged every 2 days, followed by purification according to a modification of the purification method as described in Example 1.

### Example 3

Injections of the invention was prepared as follows.

An aqueous solution was prepared aseptically by adding 1 mg of a hepatocyte growth factor and 100 mg of human serum albumin to 100 ml of 0.02 M phosphate buffer (pH 7.4) containing 0.15 M NaCl and 0.01% Polysorbate 80, and aseptically filled in a vial at 1 ml per vial, followed by lyophilization and sealing. Injectable distilled water was filled in an ampoule at 1 ml each for dissolution.

### Example 4

An aqueous solution was prepared aseptically by adding 1 mg of a hepatocyte growth factor and 100 mg of human serum albumin to 100 ml of 0.02 M phosphate buffer (pH 7.4) containing 0.15 M NaCl and 0.01% Polysorbate 80, and aseptically filled in an ampoule at 1 ml per ampoule, followed by melt-sealing.

### Example 5

An aqueous solution was prepared aseptically by adding 1 ml of a hepatocyte growth factor, 1 g of mannitol and 10 mg of Polysorbate 80 to 100 ml of physiological saline, and aseptically filled in a vial at 1 ml per vial, followed by lyophilization.

### Example 6

A solution was prepared aseptically by adding 1 mg of a hepatocyte growth factor, 1 g of mannitol and 10 mg of Polysorbate 80 to 100 ml of physiological saline, and filled in a vial at 1 ml per vial, which was then lyophilized and sealed.

### Example 7

An aqueous solution containing 1 part by weight of a hepatocyte growth factor, 50 parts by weight of human serum albumin and 100,000 parts by weight of injectable distilled water was prepared aseptically and filled in a vial, which was then lyophilized and sealed.

### Example 8

A solution was prepared aseptically by adding 1 mg of a hepatocyte growth factor, 10 mg of Polysorbate 80, 2 g of glycine and 2 g of sorbitol to 100 ml of injectable distilled water, and filled in a vial at 1 ml per vial, which was then lyophilized and sealed.

### Example 9

The growth activity of the HGF of the present invention on proximal tubular cells was confirmed as follows.

### ① Isolation of rat kidney proximal tubular cells

Wister rat was anesthetized with a barbital anesthetic and its abdomen was incised to remove kidney on an ice-cooled plate.

Renal cortex was collected and cut in small pieces, which was then subjected to Down's homogenizer. Homogenate obtained was filtered through a 245 µm nylon mesh, and further filtered through a 105 µm nylon mesh to separate renal tubular cells and granular cells, after which the fraction left on the mesh was transferred to an ice-cooled Eagle's minimum essential medium. Since a small amount of granular cells was left in this fraction, 0.01% colagenase was added to the medium, and the medium was treated at 37°C for 3 minutes, followed by removal of fibroblasts and centrifugation at 80 g for 2 minutes to give purified proximal tubular cells.

### ② Growth activity of HGF on proximal tubular cell proliferation

The thus-obtained rat HGF was added to the culture system of the proximal tubular cells extracted from rat kidney, and cell growth-promoting activity was examined in terms of increased DNA synthesis. That is, proximal tubular cells as obtained in ① were suspended in DME · F-12 mixed medium (DEM medium: ham F-12 medium=1:1, Nissui Seiyaku) supplemented with 1 × 10⁻⁸ M insulin (Sigma, USA), 1 × 10⁻⁸ M dexamethazone (Wako Junyaku), 5 µg/ml transferrin (Sigma, USA) and 5 U/ml aprotinin (Mochida Seiyaku) and seeded into a 24-well multiplate at a concentration of 4 × 10⁴ cells/well. After 24 hours' incubation at 37°C in the presence of 5% CO₂, 30% O₂ and 65% N₂, the medium was changed to DME · F-12 mixed medium supplemented with 5 U/ml aprotinin, followed by 48 hours' incubation under the same incubation conditions. With the exchange of the medium to newly-prepared DME · F-12 mixed medium supplemented with 5 U/ml aprotinin, HGF as obtained in Example 1 [added with 0.25% BSA (bovine serum albumin) and dialyzed with PBS (phosphate buffer saline)] was added as a test sample and also, a given amount of 10 ng/ml EGF (derived from male mouse submandibular gland) + 1 × 10⁻⁷ M insulin was added as positive control. After incubation for 16 hours, 1 µCi/ml of ¹²⁵I-deoxyuridine (New England Nuclear, USA) was added at 10 µl/well. Four hours later, cells were washed with PBS, placed in a 10% trichloroacetate solution and incubated for 5 minutes. The trichloroacetate was removed, cells were subjected to lysis with 1 M sodium hydroxide solution and radioactivity was measured by a gamma counter.

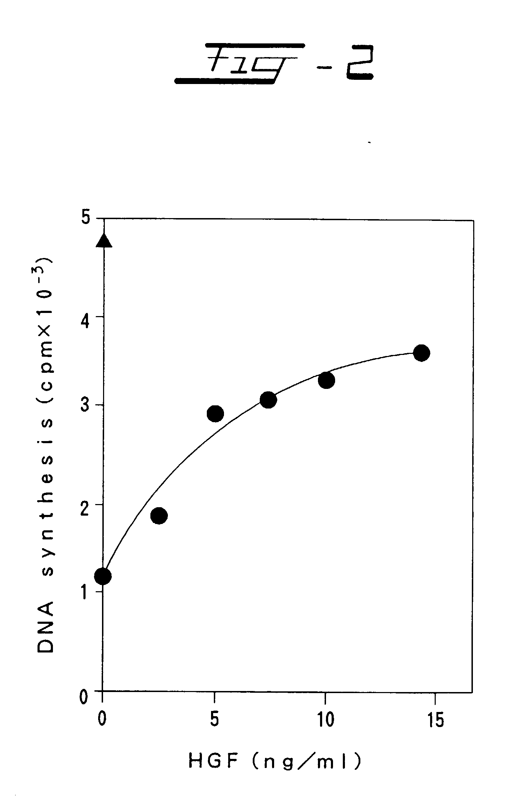

As a result, rat HGF showed dose-dependent growth-promoting activity on proximal tubular cells of rat kidney as shown in Fig. 2. That is, addition of 2 ng/ml of HGF doubled DNA synthesis of said cultured cell and that of 10 ng/ml of HGF tripled the DNA synthesis. The results confirmed that HGF which is the active ingredient of the present invention possessed growth-promoting activity on cultured nephrocytes, and renal regeneration-promoting activity in the body.

## Claims

1. Use of a hepatocyte growth factor (HGF) for the production of a therapeutic agent for renal diseases of human or mammalian animals.

2. The use according to claim 1, wherein HGF promotes nephrocyte growth.

3. The use according to claim 1, wherein the therapeutic agent promotes growth of proximal tubular cells.

4. Use of a hepatocyte growth factor (HGF) for the production of a preventive agent for renal diseases of human or mammalian animals.

5. The use according to claim 4, wherein HGF promotes nephrocyte growth.

6. The use according to claim 4, wherein the preventive agent promotes growth of proximal tubular cells.

7. The use according to any of claims 1-6, wherein the HGF is derived from human or animal tissues.

8. The use according to any of claims 1-6, wherein the HGF is produced by genetic engineering.

9. The use according to claim 8, wherein the host cell for the genetic engineering is selected from the group consisting of Escherichia coli, Bacillus subtilis, yeasts, filamentous fungi and plant and animal cells.

## Patentansprüche

1. Verwendung eines Hepatocyten-Wachstumsfaktors (HGF) zur Herstellung eines Therapeutikums für Nierenkrankheiten bei Menschen oder Säugetieren.

2. Verwendung gemäß Anspruch 1, wobei HGF das Wachstum von Nephrocyten fördert.

3. Verwendung gemäß Anspruch 1, wobei das Therapeutikum das Wachstum von Zellen proximaler Tubuli fördert.

4. Verwendung eines Hepatocyten-Wachstumsfaktors (HGF) zur Herstellung eines vorbeugenden Mittels für Nierenkrankheiten bei Menschen oder Säugetieren.

5. Verwendung gemäß Anspruch 4, wobei HGF das Wachstum von Nephrocyten fördert.

6. Verwendung gemäß Anspruch 4, wobei das vorbeugende Mittel das Wachstum von Zellen proximaler Tubuli fördert.

7. Verwendung gemäß einem der Ansprüche 1-6, wobei der HGF von menschlichen oder tierischen Geweben abgeleitet ist.

8. Verwendung gemäß einem der Ansprüche 1-6, wobei der HGF gentechnisch hergestellt ist.

9. Verwendung gemäß Anspruch 8, wobei die Wirtszelle für die gentechnische Herstellung aus der Gruppe ausgewählt ist, die aus *Escherichia coli, Bacillus subtilis,* Hefen, Fadenpilzen sowie Pflanzen- und Tierzellen besteht.

## Revendications

1. Utilisation d'un facteur de croissance des hépatocytes (HGF) pour la production d'un agent thérapeutique pour les maladies rénales chez l'homme ou les animaux mammifères.

2. Utilisation selon la revendication 1, dans laquelle HGF stimule la croissance des néphrocytes.

3. Utilisation selon la revendication 1, dans laquelle l'agent thérapeutique stimule la croissance des cellules tubulaires proximales.

4. Utilisation d'un facteur de croissance des hépatocytes (HGF) pour la production d'un agent préventif contre les maladies rénales chez l'homme ou les animaux mammifères.

5. Utilisation selon la revendication 4, dans laquelle HGF stimule la croissance des néphrocytes.

6. Utilisation selon la revendication 4, dans laquelle l'agent préventif stimule la croissance des cellules tubulaires proximales.

7. Utilisation selon l'une quelconque des revendications 1-6, dans laquelle HGF est dérivé de tissus humains ou animaux.

8. Utilisation selon l'une quelconque des revendications 1-6, dans laquelle HGF est produit par génie génétique.

9. Utilisation selon la revendication 8, dans laquelle la cellule hôte utilisée mise en oeuvre par génie génétique est choisie dans le groupe constitué par Escherichia coli, Bacillus subtilis, les levures, les champignons filamenteux et les cellules végétales et animales.
